# FASCICULE DE BREVET EUROPEEN

(11) **EP 0 387 155 B1**
(45) Date de publication et mention de la délivrance du brevet: **29.12.1993**
(21) Numéro de dépôt: 90400629.3
(22) Date de dépôt: 09.03.1990
(51) Int. Cl.: A61F 9/00

(54) **Bouchon meatique pour pathologie lacrymale**
Meatuspfropf für Lakrimalpathologie
Punctum plug for lacrymal pathology

(30) Priorité: 10.03.1989 FR 8903143
(43) Date de publication de la demande: 12.09.1990
(73) Titulaire: FRANCE CHIRURGIE INSTRUMENTATION ( S.A.R.L.), F-92134 Issy-Les Moulineaux (FR)
(72) Inventeur: Bernard, Jean-Antoine, F-75016 Paris (FR)
(74) Mandataire: Viard, Jean

(56) Documents cités:
- EP-A- 0 181 165
- FR-A- 2 632 531
- US-A- 3 949 750

## Description

La présente invention a pour objet un bouchon méatique pour pathologie lacrymale destiné en particulier, mais non exclusivement, à remédier temporairement aux kérato-conjonctivites sèches, maladies désignées dans la pratique sous le nom de "yeux secs". On sait qu'une telle maladie peut être due soit à une génération insuffisante de liquide lacrymal, soit à un drainage excessif de celui-ci par les canalicules permettant normalement d'évacuer les larmes à l'intérieur de la cavité nasale. Cette pathologie est relevée principalement chez les personnes âgées.

Il a déjà été proposé dans US-A-3 949 750 de remédier temporairement à un tel phénomène en obturant les canalicules lacrymaux nasaux au moyen d'un clou ou bouchon. Ce bouchon connu comprend une partie médiane relativement longue de diamètre inférieur à celui des deux parties terminales, la paroi interne du méat lacrymal venant s'appliquer dans la partie médiane. Le bouchon est ainsi maintenu dans le méat et ferme l'ouverture supérieure de celui-ci à l'aide d'une pièce pleine. L'extrémité opposée présente généralement une géométrie en forme de tronc de cône pour permettre une meilleure pénétration du bouchon dans le méat.

Dans la technique connue, la partie supérieure du bouchon est, d'une manière générale, perpendiculaire à l'axe du bouchon. Il en résulte que la tête du bouchon peut venir en contact avec la cornée dans certaines positions des yeux. Ce contact conduit à une irritation douloureuse. Dans certains cas, l'extrémité supérieure est hémisphérique. Mais celà conduit à des épaisseurs prohibitives.

La présente invention a pour objet de pallier cet inconvénient.

Selon la présente invention, le bouchon méatique pour pathologie lacrymale comprenant de haut en bas, un bulbe, un col et une collerette à symétrie de révolution, le diamètre de la collerette étant supérieur à celui du col et son épaisseur étant inférieure à la hauteur du bulbe est caractérisé en ce que la collerette présente une épaisseur inférieure au demi diamètre du col et est inclinée par rapport à l'axe du bouchon d'un angle d'environ 120°.

Ainsi, lors du port du bouchon dans le méat lacrymal inférieur ou supérieur, la collerette prend une position parallèle au bord de la paupière, de sorte qu'aucune irritation de la cornée ne se produise. Par ailleurs, compte tenu de la géométrie du canal, la position du bouchon est ainsi plus fermement assurée.

D'autres caractéristiques et avantages de l'invention apparaîtront au cours de la description qui va suivre d'un mode particulier de réalisation, donné uniquement à titre d'exemple non limitatif, en regard des dessins qui représentent :
- la figure 1, un schéma d'explication du problème et de la solution apportée par la présente invention ;
- la figure 2, une vue en élévation d'un bouchon selon l'invention ;
- la figure 3, une vue en perspective du même bouchon.

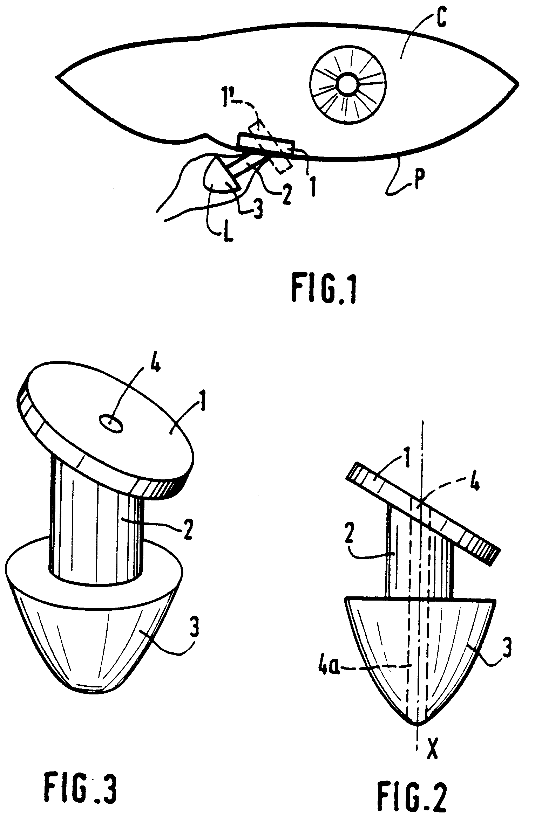

Sur la figure 1, qui représente schématiquement un oeil gauche, un bouchon méatique a été placé dans le point lacrymal inférieur, correspondant au canalicule L. On distingue sur la figure 1, la collerette 1, le col 2 et le bulbe 3 du bouchon. Conformément à l'invention, la collerette 1 du bouchon est inclinée par rapport à l'axe de celui-ci. On a représenté en 1' la position de la collerette dans le cas des bouchons méatiques connus. On comprend que les risques de contact de la cornée avec la collerette 1' sont beaucoup plus importants que ceux du contact avec la collerette 1. La collerette 1 est parallèle à la paupière P.

Comme cela paraît sur les figures 2 et 3, un bouchon comprend d'une manière générale une collerette 1, un col 2 et un bulbe 3. Conformément à l'invention, la collerette 1 est inclinée par rapport à l'axe X du bouchon méatique. Le bouchon comprend également, comme connu en soi, un trou borgne 4 de positionnement pénétrant à travers la collerette 1 dans le col 2.

Bien entendu, l'épaisseur et le diamètre de la collerette doivent être réduits au minimum afin d'éviter tout contact permanent entre celle-ci et la cornée. On comprend, à partir de la figure 1 que les bouchons selon la technique antérieure venaient en contact par un bord incliné de la collerette, ce qui produisait une irritation. En position, le disque constituant la collerette 1 est sensiblement parallèle à la paupière de sorte que, si un contact se produit, la surface de contact soit très limitée.

Bien entendu, les dimensions d'un bouchon sont déterminées par l'application recherchée et, par exemple, la collerette peut avoir un diamètre de 1,2 mm, le col une section de diamètre égal à 0,9 mm, le bulbe étant plus épais.

Le bouchon selon l'invention peut également trouver une application dans les cas d'atrésie d'un point lacrymal.

Cette pathologie provient de ce que le méat s'obstrue pour une raison quelconque et l'écoulement des larmes ne se fait plus par le canal lacrymal correspondant.

Afin de pallier cet inconvénient, le bouchon peut être percé en 4a sur toute sa hauteur (fig 2) de manière à constituer un conduit dont le diamètre est sensiblement de 0,6 mm. Ce conduit 4a, permet le lavage des voies lacrymales et son diamètre relativement important évite son obturation par des sécrétions faibles. Le conduit 4a sert également de trou de positionnement dans le méat.

## Revendications

1. Bouchon méatique pour pathologie lacrymale comprenant de haut en bas, un bulbe (3), un col (2) et une collerette (1) à symétrie de révolution, le diamètre de la collerette (1) étant supérieur à celui du col (2) et son épaisseur étant inférieure à la hauteur du bulbe (3), caractérisé en ce que la collerette (1) présente une épaisseur inférieure au demi diamètre du col (2) et est inclinée par rapport à l'axe du bouchon d'un angle d'environ 120°.

2. Bouchon selon la revendication 1, caractérisé en ce qu'il est percé de haut en bas d'un conduit axial (4a).

## Claims

1. A duct plug for tear pathology, the plug comprising from top to bottom a circularly symmetrical flange (1), a neck (2) and a bulb (3), the flange (1) being greater in diameter than the neck (2) and being of thickness less than the height of the bulb (3), the duct plug being characterized in that the flange (1) is of thickness less than half the diameter of the neck (2) and is tilted at an angle of about 120° to the axis of the plug.

2. A plug according to claim 1, characterized in that it has an axial passage (4a) running along its length.

## Patentansprüche

1. Meatuspfropf für Lakrimalpathologie, der, von oben nach unten, einen Wulstkopf (3), einen Hals (2) und einen rotationssymmetrischen Kragen (1) aufweist, wobei der Durchmesser des Kragens (1) größer als der des Halses (2) ist und seine Dicke geringer als die Höhe des Wulstkopfes (3) ist, dadurch gekennzeichnet, daß der Kragen (1) eine Dicke hat, die kleiner als der halbe Durchmesser des Halses (2) ist und in bezug auf die Achse des Pfropfens um einen Winkel von ungefähr 120° geneigt ist.

2. Propfen nach Anspruch 1, dadurch gekennzeichnet, daß er von oben nach unten mit einem axialen Kanal (4a) durchbohrt ist.
